**Europäisches Patentamt**

**European Patent Office**

**Office européen des brevets**

(19)

(11) Veröffentlichungsnummer : **0 252 241 B1**

## (12) EUROPÄISCHE PATENTSCHRIFT

(45) Veröffentlichungstag der Patentschrift :
06.02.91 Patentblatt 91/06

(51) Int. Cl.$^5$ : **C07H 15/04**

(21) Anmeldenummer : **87106743.5**

(22) Anmeldetag : **08.05.87**

---

(54) **Verfahren zur Herstellung von Butyloligoglycosiden.**

(30) Priorität : **10.07.86 DE 3623246**

(43) Veröffentlichungstag der Anmeldung :
**13.01.88 Patentblatt 88/02**

(45) Bekanntmachung des Hinweises auf die
Patenterteilung :
**06.02.91 Patentblatt 91/06**

(84) Benannte Vertragsstaaten :
**AT BE DE ES FR GB IT NL**

(56) Entgegenhaltungen :
**EP-A- 0 092 355**
**EP-A- 0 099 183**
**DE-A- 2 036 472**
**US-A- 3 375 243**

(73) Patentinhaber : **HÜLS**
**AKTIENGESELLSCHAFT**
**- RSP Patente / PB 15 - Postfach 13 20**
**D-4370 Marl 1 (DE)**

(72) Erfinder : **Lüders, Harald, Dr.**
**Langeoogstrasse 81**
**D-4350 Recklinghausen (DE)**

## Beschreibung

Butyloligoglycoside oder Gemische aus Butylglycosiden und Butyloligoglycosiden sind interessante Polyole, die über Kohlenhydrate zuganglich sind. Sie erlangen als Zwischenprodukte bei der Herstellung von Polyurethanen und von Tensiden auf der Basis langkettiger Alkylglycoside zunehmende Bedeutung.

Alkylglycoside mit kurzkettigen Alkylgruppen werden im allgemeinen nach dem Fischer-Verfahren hergestellt. So werden nach US-PS 3 974 138 Butylglucoside aus Glucose und Butanol unter Saurekatalyse durch Erhitzen bis zum Rückfluß erhalten. Bei diesem Verfahren muß Glucose in kristalliner Form eingesetzt werden. Billigere, wäßrige Glucose- bzw. Dextrosesirupe können nicht eingesetzt werden, da sich Glucose in Form zaher, klebriger Massen aus dem Reaktionsgemisch abscheidet, sobald das zur Hydrolyse Anlaß gebende Wasser abdestilliert wird.

In DE-OS 20 36 472 wird ein Verfahren zur Herstellung von Alkyloligoglucosiden aus Sacchariden und einwertigen Alkoholen mit 8 bis 25 C-Atomen unter Saurekatalyse beschrieben. Bei diesem Verfahren werden Glykole mit 3 bis 5 C-Atomen zugesetzt, um Sirupzucker einsetzen zu können. Die Glykole fungieren als Losungsmittel sowohl für die Alkohole als auch für die Zucker. Nach diesem Verfahren können reine Butylglycoside nicht hergestellt werden. Die Glykole haben nämlich einen höheren Siedepunkt als Butanol und könnten deshalb in Gegenwart des Butanols während der Reaktion nicht abdestilliert werden. Im Reaktionsprodukt würden Glykolglycoside verbleiben.

In EP 0 099 183 werden Alkylglycoside aus wasserhaltigen Polysacchariden und Alkoholen mit 3 bis 25 C-Atomen säurekatalysiert hergestellt. Die Reaktion wird in Gegenwart von Cosolventien wie Methanol, Ethanol, Ethylenglykol oder Aceton und unter Druck durchgeführt. Das Reaktionsprodukt erfordert zur Reinigung einen erhöhten Destillationsaufwand, und es enthält einen hohen Gehalt an unumgesetzter Glucose.

Nach EP 0 092 355 werden Alkylglucosidmischungen aus Glucose und langkettigen Alkoholen mit 8 bis 22 C-Atomen säurekatalysiert hergestellt. Dabei wird eine Verträglichkeit der Ausgangsstoffe durch nichtionische Tenside erreicht. Als derartige Tenside werden Alkylglycoside mit Alkylgruppen mit mindestens 8 C-Atomen, vorzugsweise mit 10 bis 14 C-Atomen, zugesetzt. Da bei der Herstellung von Tensiden des Alkyloligoglucosid-Typs im allgemeinen Fettalkoholgemische verwendet werden, wird die Produktqualität durch Zusatz eines im Alkylrest abweichenden Tensids praktisch nicht beeinflußt. Bei der Herstellung von Alkyloligoglycosiden mit einer speziellen Alkylgruppe mit weniger als 8 C-Atomen wird dagegen ein derartiges Tensid das Endprodukt kontaminieren, da durch Umglycosidierung freigesetzte Fettalkohole in Gegenwart kurzkettiger Alkohole destillativ nicht abzutrennen sind.

Aufgabe der vorliegenden Erfindung ist es, ein vereinfachtes Verfahren zur Herstellung von Butyloligoglycosiden bereitzustellen, bei dem billige, wäßrige Saccharidsirupe als Ausgangsstoffe eingesetzt werden können, ohne daß dabei die Handhabbarkeit der Reaktionsmischung durch Inhomogenitäten oder durch Ausfällung von klebrigen Zuckermengen erschwert wird.

Überraschenderweise wird die Aufgabe dadurch gelöst, daß man wäßrige Saccharidsirupe bei erhöhter Temperatur in Gegenwart von Butyloligoglycosiden, die nicht als Tenside anzusehen sind, mit Butanol umsetzt und während der Reaktion Wasser azeotrop abdestilliert.

Für die Reaktion können Saccharidsirupe auf der Basis von Hexosen wie Glucose, Mannose, Galaktose, Sorbose oder Fructose, von Pentosen wie Xylose, Arabinose, Ribose oder Lyxose sowie von Disacchariden wie Lactose, Maltose oder Saccharose und auf der Basis von deren Mischungen eingesetzt werden. Vorzugsweise setzt man handelsübliche Stärkehydrolysatsirupe wie Dextrose- und Glucosesirupe oder Maltodextrinsirupe ein. Die Produkte können auch Oligosaccharide enthalten. Der DE-Wert (Dextrosewert) sollte jedoch über 20 liegen. Die Wassergehalte der Produkte liegen im allgemeinen bei 20 bis 40%. Vorzugsweise verwendet man Produkte mit Wassergehalten von 25 bis 30%.

In der Reaktionsmischung werden pro Saccharideinheit der Saccharidsirupe 1 bis 20 Mol Butanol eingesetzt. Gleichzeitig sorgt man dafür, daß pro Saccharideinheit der zugesetzten Butyloligoglycoside 1 bis 10 Mol Butanol vorhanden sind.

Als Katalysatoren dienen Mineralsäuren, starke organische Säuren oder saure Ionenaustauscher. Bevorzugt wird mit Schwefelsäure oder mit p-Toluolsulfonsäure katalysiert. Die Katalysatoren werden in Konzentrationen von 0,01 bis 1,0%, bezogen auf die Reaktionsmischung, eingesetzt.

Die Reaktion wird bei 80 bis 130°C durchgeführt. Vorzugsweise liegt die Temperatur bei 100 bis 120°C. Bei Temperaturen unter 80°C ist die Reaktionsgeschwindigkeit gering. Oberhalb 130°C wird die Bildung von Nebenprodukten stärker begünstigt und die Farbe der Produkte verschlechtert.

Durch den Zusatz von Butyloligoglycosiden kann die Reaktion in einer homogenen Lösung durchgeführt werden. Die benötigten Butyloligoglycosidmengen sind im wesentlichen von der Reaktionstemperatur und dem Wassergehalt der Sirupe abhängig. So wird beispielsweise aus folgender Mischung bei 100°C eine homogene Lösung :

30 Gew.-% Butanol
40 Gew.-% Butylglucosid
30 Gew.-% Glucosesirup mit 30% Wasser

Für den diskontinuierlichen Betrieb können als Reaktoren Rührkolben mit einer Einrichtung zum Auskreisen von Wasser verwendet werden. Für den kontinuierlichen Betrieb eignen sich unter anderem Rührkesselkaskaden, bei denen mit Hilfe von Wasserabscheidern Wasser abgetrennt werden kann.

Bei einem diskontinuierlichen Ansatz kann wie folgt verfahren werden :

Man legt zunächst in einem Rührkolben Butanol und Butyloligoglucosid vor. Nach Zusatz von Säure als Katalysator wird zum Sieden erhitzt, wobei ggf. Überdruck oder leichtes Vakuum angewandt wird, um eine Temperatur von 80 bis 130°C einzustellen. Nun werden Butanol, wäßriger Saccharidsirup sowie weitere Säure zudosiert. Gleichzeitig wird Wasser über einen Wasserabscheider abgetrennt. Die Dosiergeschwindigkeit der Edukte wird so abgestimmt, daß in der Reaktionsmischung Butyloligoglucosid in einer Konzentration vorliegt, durch die Inhomogenitäten ausgeschlossen werden können. Am Ende der Reaktion kann mit einer Lauge neutralisiert und überschüssiges Butanol abdestilliert werden.

Bei kontinuierlicher Fahrweise wird Butyloligoglycosid der Reaktionsmischung zunächst zugesetzt, worauf während der Reaktion eine Konzentration von mindestens 15% in der Mischung aufrechterhalten wird und Wasser abgetrennt wird. Vorzugsweise beträgt die Butyloligoglycosidkonzentration mindestens 20%.

Das Verfahren weist folgende Vorteile auf :

1. Es können billige, wäßrige Saccharidsirupe verarbeitet werden.

2. Alle Komponenten, die während des Prozesses dosiert werden, sind flüssig und leicht pumpbar.

3. Das Reaktionsgemisch bleibt während des gesamten Prozesses homogen.

4. Das Verfahren kann in einfacher Weise in einer Rührkesselkaskade mit Wasserabscheidern kontinuierlich durchgeführt werden.

5. Ein Zusatz von später zu entfernenden Hilfsstoffen ist nicht erforderlich.

Beispiel 1

In einer Rührkolbenkaskade aus drei 8 l-Rührkolben, die mit Destillationskolonnen und Wasserabscheidern ausgerüstet sind, wird eine Lösung aus 7,5 kg Butyloligoglucosid (mittlerer Oligomerisationsgrad ca. 1,8), 7,5 kg n-Butanol und 63 ml 2 N Schwefelsäure gleichmäßig über die drei Kolben verteilt. Die Kolben werden dann beheizt, so daß die Lösung unter Rückfluß kocht.

Nun werden in den ersten Kolben 1728 g/Std. 70%iger wäßriger Dextrosesirup, 2240 g/Std. n-Butanol und 33,6 ml/Std. 2 N Schwefelsäure zudosiert. Die Reaktionstemperatur liegt bei 115 bis 120°C. Über die Wasserabscheider werden etwa 830 g/Std. Wasserphase ausgekreist.

Aus dem letzten Kolben werden 3 190 g/Std. Produktlösung abgetrennt. Die Füllstandshöhen in den Kolben bleiben dabei konstant. Die Produktlösung besteht zu ca. 45% aus Butyloligoglucosid und enthält unter 1% unumgesetzte Glucose.

Nach Neutralisation mit 2 N Natronlauge wird n-Butanol abdestilliert. Als Sirup isoliert man ein Butyloligoglucosid mit einem mittleren Oligomerisationsgrad von ca. 1,8.

Beispiel 2

In einer Rührapparatur mit Destillationskolonne und Wasserabscheider werden 100 g Butylglucosid, 108 g 70%iger Glucosesirup und 62 g n-Butanol auf 110°C erhitzt. 2,4 ml 2 N Schwefelsäure werden zugefügt. Anschließend wird 4 Stunden zum Sieden erhitzt und dabei Wasser ausgekreist.

Nach Neutralisation mit 2 N Natronlauge wird eingedampft, wobei Butylglucosid als Sirup erhalten wird.

**Ansprüche**

1. Verfahren zur Herstellung von Butyloligoglycosiden durch säurekatalysierte Umsetzung von Sacchariden mit Butanol bei erhöhter Temperatur, dadurch gekennzeichnet,
daß man
– wäßrige Saccharidsirupe einsetzt,
– Butyloligoglycoside zusetzt und
– Wasser während der Reaktion azeotrop abdestilliert.

2. Verfahren nach Anspruch 1, dadurch gekennzeichnet,
daß in der Reaktionsmischung das Molverhältnis von Butanol zu Saccharideinheit der Saccharidsirupe 1 : 1 bis 20 : 1 beträgt.

3. Verfahren nach Anspruch 1, dadurch gekennzeichnet,
daß das Molverhältnis von Butanol zu Saccharideinheit der zugesetzten Butyloligoglycoside 1 : 1 bis 10 : 1 beträgt.

4. Verfahren nach den Ansprüchen 1 bis 3, dadurch gekennzeichnet,
daß man Saccharidsirupe mit Wassergehalten von 20 bis 40% einsetzt.

5. Verfahren nach Anspruch 4, dadurch gekennzeichnet,
daß man Dextrosesirupe mit 25 bis 30% Wasser einsetzt.

6. Verfahren nach den Ansprüchen 1 bis 5, dadurch gekennzeichnet,
daß man die Umsetzung durch Schwefelsäure oder p-Toluolsulfonsäure katalysiert.

7. Verfahren nach den Ansprüchen 1 bis 6, dadurch gekennzeichnet,

daß die Reaktion bei 80 bis 130°C durchgeführt wird.

8. Verfahren nach Anspruch 7,
dadurch gekennzeichnet,
daß die Reaktion bei 100 bis 120°C durchgeführt wird.

9. Verfahren nach den Ansprüchen 1 bis 8,
dadurch gekennzeichnet,
daß man die Reaktion in einer kontinuierlichen Rührkesselkaskade durchführt, Butyloligoglycoside zunächst zusetzt und dann in einer Konzentration von mindestens 15% in der Reaktionsmischung hält und Wasser kontinuierlich abtrennt.

10. Verfahren nach Anspruch 9,
dadurch gekennzeichnet,
daß die Butyloligoglycosidkonzentration mindestens 20% beträgt.

## Claims

1. A process for the preparation of butyl oligoglycosides by acid-catalysed reaction of saccharides with butanol at elevated temperature, characterised in that
    – aqueous saccharide syrups are used,
    – butyl oligoglycosides are added, and
    – water is distilled off azeotropically during the reaction.

2. A process according to claim 1, characterised in that the molar ratio of butanol to saccharide unit of the saccharide syrups in the reaction mixture is 1 : 1 to 2 : 1.

3. A process according to claim 1, characterised in that the molar ratio of butanol to saccharide unit of the butyl oligoglycosides added is 1 : 1 to 10 : 1.

4. A process according to any of claims 1 to 3, characterised in that saccharide syrups having water contents of 20 to 40% are used.

5. A process according to claim 4, characterised in that dextrose syrups containing 25 to 30% of water are used.

6. A process according to any of claims 1 to 5, characterised in that the reaction is catalysed by sulphuric acid or p-toluenesulphonic acid.

7. A process according to any of claims 1 to 6, characterised in that the reaction is carried out at 80 to 130°C.

8. A process according to claim 7, characterised in that the reaction is carried out at 100 to 120°C.

9. A process according to any of claims 1 to 8, characterised in that the reaction is carried out in a continuous stirred boiler cascade, the butyl oligoglycosides are first added and then maintained at a concentration of at least 15% in the reaction mixture, and water is separated off continuously.

10. A process according to claim 9, characterised in that the butyl oligoglycoside concentration is at least 20%.

## Revendications

1. Procédé de préparation de butyl-oligo-glycosides en faisant réagir, en présence d'un catalyseur acide, des saccharides sur du butanol à température élevée, caractérisé par le fait :
    – que l'on utilise des sirops aqueux de saccharide,
    – que l'on ajoute des butyl-oligo-glycosides, et
    – que l'on élimine l'eau par distillation azéotropique pendant la réaction.

2. Procédé selon la revendication 1, caractérisé par le fait que, dans le mélange réactionnel, la proportion molaire du butanol par rapport à l'unité saccharide du sirop de saccharide est de 1 : 1 à 20 : 1.

3. Procédé selon la revendication 1, caractérisé par le fait que la proportion molaire du butanol par rapport à l'unité saccharide des butyl-oligo-glycosides ajoutés est de 1 : 1 à 10 : 1.

4. Procédé selon les revendications 1 à 3, caractérisé par le fait que l'on utilise des sirops de saccharide présentant des teneurs en eau de 20 à 40%.

5. Procédé selon la revendication 4, caractérisé par le fait que l'on utilise des sirops de dextrose renfermant de 25 à 30% d'eau.

6. Procédé selon les revendications 1 à 5, caractérisé par le fait que l'on catalyse la réaction par l'acide sulfurique ou l'acide p-toluène-sulfonique.

7. Procédé selon les revendications 1 à 6, caractérisé par le fait que l'on effectue la réaction à une température de 80 à 130°C.

8. Procédé selon la revendication 7, caractérisé par le fait que l'on effectue la réaction à une température de 100 à 120°C.

9. Procédé selon les revendications 1 à 8, caractérisé par le fait que l'on effectue la réaction dans une cascade continue de récipients d'agitation, que l'on ajoute d'abord les butyl-oligo-glycosides et qu'on les maintient ensuite dans le mélange réactionnel dans une concentration de 15% au moins et qu'on sépare l'eau en continu.

10. Procédé selon la revendication 9, caractérisé par le fait que la concentration en butyl-oligo-glycoside est de 20% au moins.